# NEUE EUROPÄISCHE PATENTSCHRIFT

(11) **EP 0 634 212 B2**
(45) Veröffentlichungstag und Bekanntmachung des Hinweises auf die Entscheidung über den Einspruch: **16.12.1998**
(45) Hinweis auf die Patenterteilung: 12.06.1996
(21) Anmeldenummer: 94110328.5
(22) Anmeldetag: 02.07.1994
(51) Int. Cl.: B01J 29/89, C07C 249/08, C07D 303/04

(54) **Strukturierter Katalysator, bestehend aus mikroporösen Oxiden von Silicium, Aluminium und Titan**
Structured catalyst, containing microporous oxides of silicium, aluminium and titanium
Catalyseur structuré, comprenant des oxides microporeux d'aluminium, de silicium et de titanium

(30) Priorität: 12.07.1993 DE 4323255; 01.06.1994 DE 4419195
(43) Veröffentlichungstag der Anmeldung: 18.01.1995
(73) Patentinhaber: Degussa Aktiengesellschaft, 60311 Frankfurt (DE)
(72) Erfinder: Thiele, Georg, Dr., D-63452 Hanau (DE); Roland, Eckehart, Dr., D-63486 Bruchköbel (DE)

(56) Entgegenhaltungen:
- EP-A- 0 055 044
- EP-A- 0 118 632
- EP-A- 0 200 260
- EP-A- 0 208 311
- EP-A- 0 311 983
- EP-A- 0 543 247
- DE-A- 3 047 798
- Applied Catalysts A: General, 96 (1993), 151-161, "Alkylation of toluene over double structure ZSM-5 type catalysts covered with a silicalite shell"
- A. Thangarai et al., J.Catal. 131 (1991), 394
- G.Belussi et al., Stud. Surf. Sci. Cat., Preparation of Catalysts V, 1991, 421-429 "Double substitution in silicalite by direct synthesis: A new route to crystalline porous bifunctional catalysts"

## Beschreibung

Die Erfindung betrifft einen strukturierten Katalysator aus mikroporösen Oxiden von Silicium, Aluminium und Titan sowie ein Verfahren zur Herstellung dieses Katalysators.

Mikroporöse Alumosilicate der Strukturtypen MFI (ZSM-5) und MEL (ZSM-11) sowie Verfahren zu deren Herstellung durch Hydrothermalsynthese sind aus US 3,702,886 bzw. DE 21 19 723 bekannt. Die Strukturtypen MFI und MEL sind in W. M. Meier, D. H. Olson, Atlas of Zeolite Structure Types, Butterworth-Heinemann, 1993, beschrienen. Aluminiunfreie Materialien der gleichen Strukturen, bekannt als Silicalit-1 und Silicalit-2, sind in US 4,073,865, US 4,061,724 bzw. D.M. Bibby, N.B. Milestone, L.P. Aldridge, Nature 280, 664 (1979), EP 112 006 offenbart. Materialien, bei denen in den Strukturen von Silicalit-1 oder Silicalit-2 ein Teil der Silicum-Atome durch Titan-Atome ersetzt ist, sind als Titansilicalite TS-1 und TS-2 bekannt und in DE 30 47 798 und J.S. Reddy, R. Kumar, P Ratnasamy, Appl. Catal. 58 (1990) L1 - L4 beschrieben. Die Titansilicalite TS-1 und TS-2 sind wirksame Katalysatoren für selektive Oxidationen mit Wasserstoffperoxid, vor allem für die Epoxidierung von Olefinen (EP 100 119), Hydroxylierung von Aromaten (DE 33 09 669 und J.S.Reddy, R. Kumar, P. Ratnasamy, Appl. Catal. 58 (1990) L1 - L4), Hydroxylierung von Aliphaten (EP 412 596 und J.S. Reddy, S. Sivasanker, P. Ratnasamy, J. Mol. Catal. 70 (1991) 335 - 342) und die Ammoximierung von Cyclohexanon (EP 208 311 und J.S. Reddy, S. Sivasanker, P. Ratnasamy. J. Mol. Catal. 69 (1991) 383 - 392).

Die bekannte Herstellung der Titansilicalite TS-1 und TS-2 erfolgt durch eine zweistufige Synthese. Zunächst wird ein Gel durch Hydrolyse einer Titan-Quelle wie TiCl₄, TiOCl₂, Ti(OAlkyl)₄, zumeist Ti(OAlkyl)₄ und einer Silicium-Quelle wie Kieselgel, Si(OAlkyl)₄, zumeist Si(OAlkyl)₄, hergestellt. Anschließend wird dieses Gel in einer Hydrothermalsynthese durch Erhitzen unter Druck kristallisiert, wobei zur Kristallisation ein Templat zugesetzt werden muß, wie Tetra-n-propylammoniumhydroxid für TS-1 oder Tetra-n-butylammoniumhydroxid für TS-2. Der hohe Preis von Ti(OAlkyl)₄, Si(OAlkyl)₄ und des Templats trägt wesentlich zu den Kosten für die Herstellung von TS-1 und TS-2 bei.

Die Titansilicalite TS-1 und TS-2 werden bei dem bekannten Verfahren meistens in Form kleiner Kristallite von weniger als einem Mikrometer Größe gebildet, die sich von Flüssigkeiten nur schwer durch Filtration abtrennen lassen. Für eine technische Anwendung dieser Materialien ist deshalb ein zusätzlicher Agglomerationsschritt erforderlich. Ein Beispiel für eine solche Agglomeration ist in EP 203 260 beschrieben.

Bei der Anwendung der Titansilicalite TS1 und TS2 als Katalysatoren für Oxidationsreaktionen mit Wasserstoffperoxid wird die katalytische Aktivität durch die Molekülgröße und Molekülgestalt der zu oxidierenden Verbindung bestimmt (M. Clerici, P. Ingallina, J. Catal. 140 (1993) 71 - 83). Daraus läßt sich auf eine Begrenzung der katalytischen Aktivität durch den Stofftransport in den Hohlräumen des Kristallgitters schließen, so daß Titan-Atome im Inneren des Kristalls weniger zur katalytischen Aktivität beitragen als Titan-Atome nahe der Oberfläche des Kristalls.

Es besteht deshalb ein Bedarf an Katalysatoren, die eine ähnliche Aktivität wie Titansilicalite in selektiven Oxidationsreaktionen mit Wasserstoffperoxid zeigen und die sich mit geringeren Mengen an Ti(OAlkyl)₄ , Si(OAlkyl)₄, und Templat herstellen lassen und eine gezielte Einstellung der Kristallitgröße erlauben und damit eine bessere Nutzung der katalytischen Aktivität der Titanatome erlauben.

Es ist bekannt, Katalysatorkompositionen herzustellen, die aus einem Siliciumdioxidkern und einem daraufgeschicheten modifizierten Zeolith bestehen. Der Siliciumdioxidkern besteht aus aluminiumfreien Silicaliten, die zum Beispiel die gleiche Struktur wie ZSM-5 aufweisen.

Die auf den Kern aufgebrachte Schicht kann ein mit Titan modifizierter Zeolith des Typs ZSM-5 sein. Die bekannte Katalysatorkomposition kann zum Beisiel zur Xylolumlagerung eingesetzt werden (EP-A 0 055 044).

Es ist bekannt, Titansilikalitkristralle zu agglomerieren, indem man die Titansilikatlitkristalle mit einem oligomeren Siliciumdioxid verbindet (EP-A 0 200 260).

Gegenstand der Erfindung ist ein Katalysator, bestehend aus den Oxiden von Silicium, Aluminium und Titan, welcher dadurch gekennzeichnet ist, daß die Katalysatorteilchen aus einem Kern der Zusammensetzung (SiO₂)ₓ(AlO₂)_{y}M_{y} mit x/y = 10-75 und M = H, Na, K, NH₄, NR₄, wobei R = C 1-8-Alkyl ist, und einer Schale der Zusammensetzung (SiO₂)ₙ(TiO₂)ₘ mit n/m = 12 - 1000 aufgebaut sind und sowohl Kern als auch Schale eine Kristallstruktur vom Typ MFI oder MEL besitzen.

In einer besonderen Ausführungsform weist die Schale des Katalysators die Zusammensetzung (SiO₂)ₙ(TiO₂)ₘ mit n/m = 20 - 200 auf.

Der erfindungsgemäße Katalysator kann hergestellt werden, indem man auf bekanntem Wege ein Synthesegel für die Herstellung eines Titansilicalits herstellt, wobei eine Titan-Quelle wie TiCl₄, TiOCl₂, Ti(OAlkyl)₄ und eine Siliciumquelle wie Kieselgel oder Si(OAlkyl)₄ gemeinsam hydrolysiert werden können und ein Tetraalkylammoniumhydroxid als Templat zusetzt werden kann, in dieses Synthesegel ein kristallines Alumosilicat, zum Beispiel des Strukturtyps MFI oder MEL, zum Beispiel Zeolith ZSM-5 bzw. ZSM-11, einbringt und das Synthesegel auf bekanntem Wege, zum Beispiel durch Kristallisation unter hydrothermalen Bedingungen und Abtrennen, Filtrieren und Calcinieren des kristallinen Produkts aufarbeitet.

Das kristalline Alumosilicat kann bereits vorder Gelbildung, während der Phase der Gelbildung den Rohstoffen oder aber dem fertigen Gel vor der Kristallisation zugesetzt werden. Bei der Zugabe des Alumosilicats in der protonierten H-Form (H-ZSM-5 bzw. H-ZSM-11) kann dieses als saure Komponente die Präkondensation einleiten.

Die Zusammensetzung des Synthesegels kann im Bereich der Molverhältnisse
SiO₂/TiO₂ = 5 - 1000
OH⁻/SiO₂ = 0,1 - 1,0
H₂O/SiO₂ = 15 - 200, vorzugsweise 20 bis 200
RN₄⁺/SiO₂ = 0,1 - 2,0
gewählt werden.

Das Verhältnis zwischen der Menge des in dem Synthesegel enthaltenen SiO₂ und TiO₂ und der Menge des dem Synthesegel zugesetzten kristallinen Alumosilicats kann im Bereich eines Massenverhältnisses von 0.02 bis 20 gewählt werden.

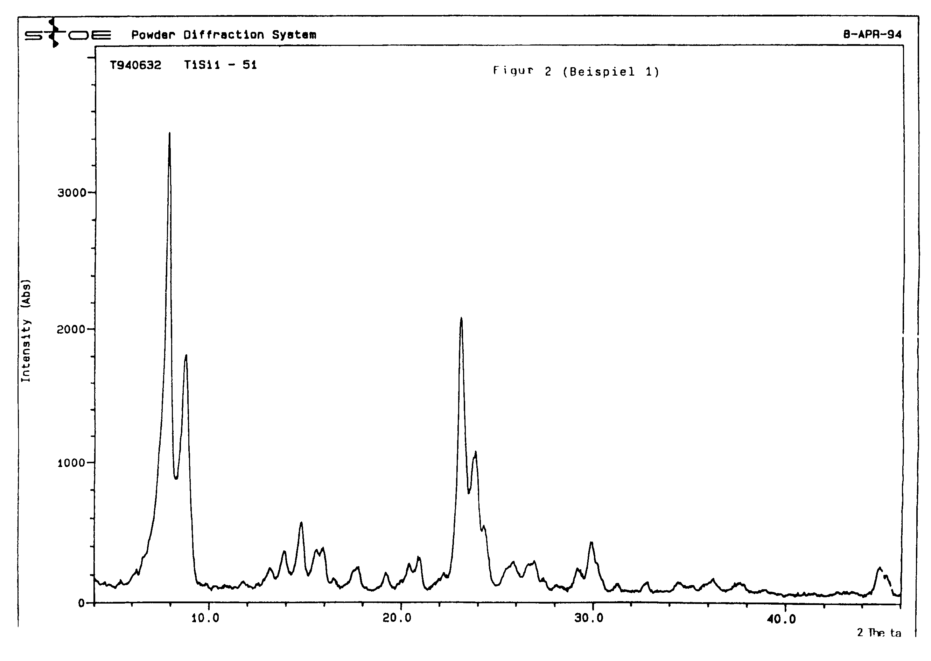

Die auf diesem Weg hergestellten erfindungsgemäßen Katalysatorteilchen zeigen in der rasterelektronenmikroskopischen Aufnahme die gleiche Morphologie und Partikelgröße wie das als Kernmaterial zugesetzte Alumosilicat. Ihr mittlerer Teilchendurchmesser ist größer als der von Titansilicalit, der in Abwesenheit des Kernmaterials hergestellt wurde. Im Röntgendiffraktogramm (Figuren 2 bis 5) zeigen sie die für kristalline Zeolithe vom Strukturtyp MFI charakteristischen Reflexe. Die im IR-Spektrum bei 960-975 cm⁻¹ beobachtete Bande beweist den Einbau von isolierten Titanatomen in das Kristallgitter des Materials.

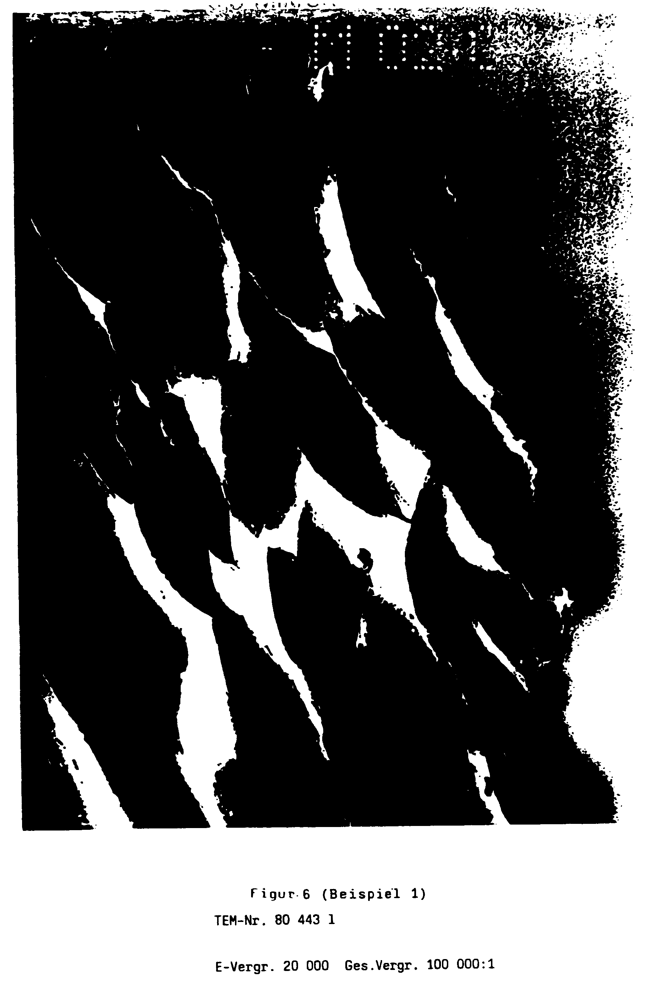

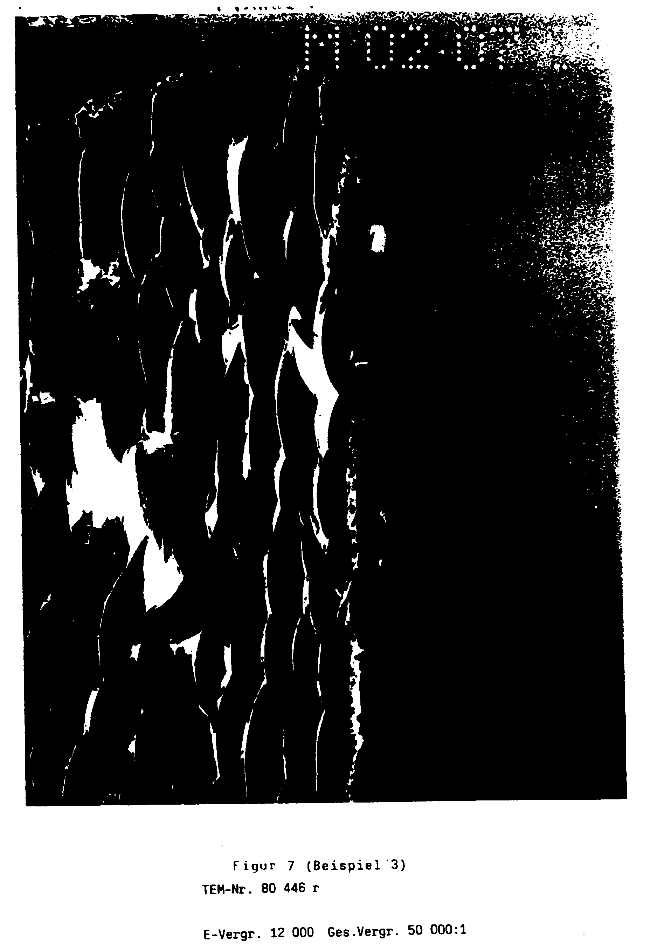

Transmissionselektronenmikroskopische Aufnahmen von Schnitten der erfindungsgemäßen Katalysatorteilchen (Figuren 6 und 7) zeigen den Aufbau der Katalysatorteilchen aus einer geschlossenen Schale auf dem eingesetzten Kernmaterial. Die Röntgen-Photoelektronen-Spektroskopie (XPS) der Katalysatorteilchen zeigt, daß die Oberfläche der Katalysatorteilchen nur Titan und Silicium, jedoch kein Aluminium enthält. Beim Abtragen eines Teils der Katalysatoroberfläche durch Sputtern nimmt der durch XPS nachgewiesene Titangehalt deutlich ab und es wird das im Kern der Teilchen enthaltene Aluminium nachgewiesen. Dies beweist den Aufbau der erfindungsgemäßen Katalysatoren aus einem Kern, der im wesentlichen die Zusammensetzung des bei der Herstellung des Katalysators eingesetzten Alumosilicats aufweist, und einer Schale der Zusammensetzung (SiO₂)ₙ(TiO₂)ₘ.

Die Erfindung hat gegenüber dem bekannten Stand der Technik den Vorteil, daß im Katalysator die katalytisch aktiven Titan-Atome gezielt in eine Schicht an der Oberfläche des Kristalls eingebaut sind, wobei die Dicke dieser Schicht durch die Wahl der Herstellbedingungen gesteuert werden kann. Durch diese Vorgehensweise kann gegenüber dem Stand der Technik der Bedarf an teuren Einsatzstoffen gesenkt werden. Durch die Verwendung von Ausgangskristallen definierter Größe können ohne zusätzlichen Agglomerationsschritt größere Katalysatorteilchen, deren Größe über die Wahl der Größe der Ausgangskristalle und die Wahl der Synthesebedingungen vorherbestimmt werden kann, hergestellt werden.

Der erfindungsgemäße Katalysator kann zur selektiven Oxidation mit H₂O₂ in flüssiger Phase, zum Beispiel zur Ammoximierung von Ketonen, wie z.B. Cyclohexanon oder Cyclododecanon, oder zur Epoxidierung von Olefinen, wie z.B. Propen, 1-Buten, 2-Buten, 1-Penten, Allylchlorid oder Allylalkohol, eingesetzt werden.

Für die Verwendung zur Herstellung von säureempfindlichen Produkten, wie z.B. Epoxiden oder Cyclohexanonoximen, kann der Katalysator nach dem Calcinieren durch die Behandlung mit einer Base mit einem pK_{B}-Wert zwischen 0 und 11, vorzugsweise einer wäßrigen Lösung von Natriumacetat, Natriumcarbonat, Natriumbicarbonat oder Ammoniak, neutralisiert werden, sodaß eine wäßrige Suspension des Katalysators nach der Neutralisation einen pH-Wert zwischen 5 und 9 aufweist.

### Beispiele

### Herstellung der Katalysatoren

### Beispiel 1:

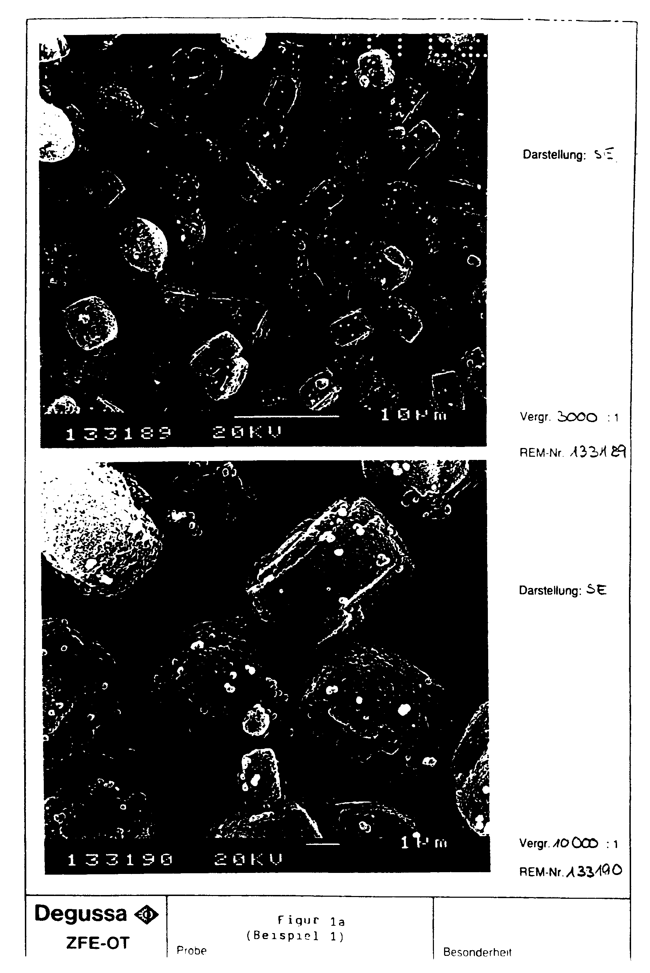

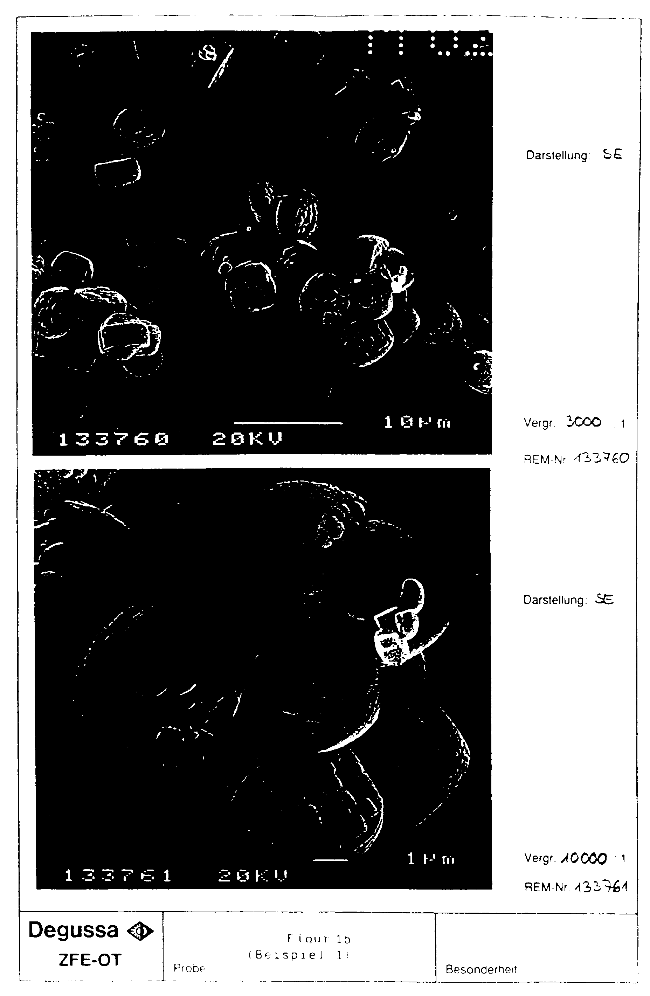

In einem Rührkolben (500 ml), der mit Rührer, Thermometer und Rückflußkühler versehen ist und bei dem der Zutritt von Luftfeuchte durch ein CaCl₂-Rohr ausgeschlossen wird, werden 37,2 ml Si(OEt)₄ mit 44,4 ml absolutem i-Propanol verdünnt. Dann werden unter Rühren 2,8 ml Ti(On-Bu)₄ in 8,5 ml absolutem i-Propanol bei Raumtemperatur zugetropft. Schließlich werden 23,8 g H-ZSM-5 (SiO₂/Al₂O₃ = 67, d₅₀ = 7.6 µm), der vorher 1 h bei 550°C calciniert wurde, zugesetzt. Die entstandene Suspension wird 2 h unter Rückfluß erhitzt. Nach Abkühlen auf Raumtemperatur werden innerhalb von 10 min 42,5 ml einer 20 Gew.-% wäßrigen Lösung von Tetra-n-propylammoniumhydroxid zugetropft, wobei sich die Mischung leicht erwärmt. Anschließend wird der Kolben mit einer Destillationsbrücke versehen. Das Gemisch der Alkohole-Propanol, Ethanol und n-Butanol und etwas Wasser werden abdestilliert. Der resultierende Rückstand wird mit 86 ml Wasser versetzt und aufgerührt. Diese Mischung wird zur Kristallisation in einen mit Rührer versehenen und mit Teflon ausgekleideten Autoklaven (500 ml) überführt und 22 h auf 180°C erhitzt. Der erhaltene Feststoff wird durch Zentrifugieren isoliert, zweimal mit je 50 ml destilliertem Wasser gewaschen, bei 120°C getrocknet und 10 h bei 550°C calciniert. Anschließend wird das Produkt 1 h bei 80°C mit 150 ml einer 10 Gew.-% wäßrigen Ammoniumacetat-Lösung behandelt, abzentrifugiert, zweimal mit je 50 ml destilliertem Wasser gewaschen, bei 120°C getrocknet und 10 h bei 550°C calciniert.
Naßchemisch bestimmte Zusammensetzung des so hergestellten Katalysators: TiO₂: 1,6 Gew.-%; SiO₂: 96,8 Gew.-%; Al₂O₃: 1,6 Gew.-%.
Das Röntgendiffraktrogramm (Figur 2) zeigt, daß der Katalysator aus kristallinem Material vom Strukturtyp MFI besteht.
Das IR-Spektrum zeigt eine Schulter, die charakteristisch für den Einbau von isolierten Titanatomen in das Kristallgitter des Materials ist, bei 965 cm⁻¹.
Die rasterelektronenmikroskopische Aufnahme der Katalysatorteilchen (Figur 1a, Vergrößerung 3000:1 und 10.000:1) zeigt im Vergleich mit der rasterelektronenmikroskopischen Aufnahme des eingesetzten H-ZSM-5-Kernmaterials (Figur 1b, Vergrößerung 3000:1 und 10.000:1), daß bei der Herstellung des Katalysators die Morphologie und Partikelgröße des Kernmaterials im wesentlichen erhalten bleibt.
Das transmissionselektronenmikroskopisches Schnittbild eines Katalysatorteilchens (Figur 6, Vergrößerung 100.000:1) zeigt den Aufbau aus einer 0.08-0.15 µm dicken, geschlossenen Schale auf dem Kernmaterial.
Die mit Röntgen-Photoelektronen-Spektroskopie (XPS) bestimmte Oberflächenzusammensetzung (Si: 30,8 Mol-%; Ti: 0,48 Mol-%; Al nicht nachweisbar) belegt, daß die Schale des Katalysators aus aluminiumfreiem Titansilicalit besteht. Die nach Abtragen der Oberfläche durch Sputtern gefundene Oberflächenzusammensetzung (Si: 34,7 Mol-%; Ti: 0,19 Mol-%; Al: 0,80 Mol-%) belegt, daß das Titan im wesentlichen nur in der Schale und nicht im Kern des Katalysators enthalten ist.

### Beispiel 2:

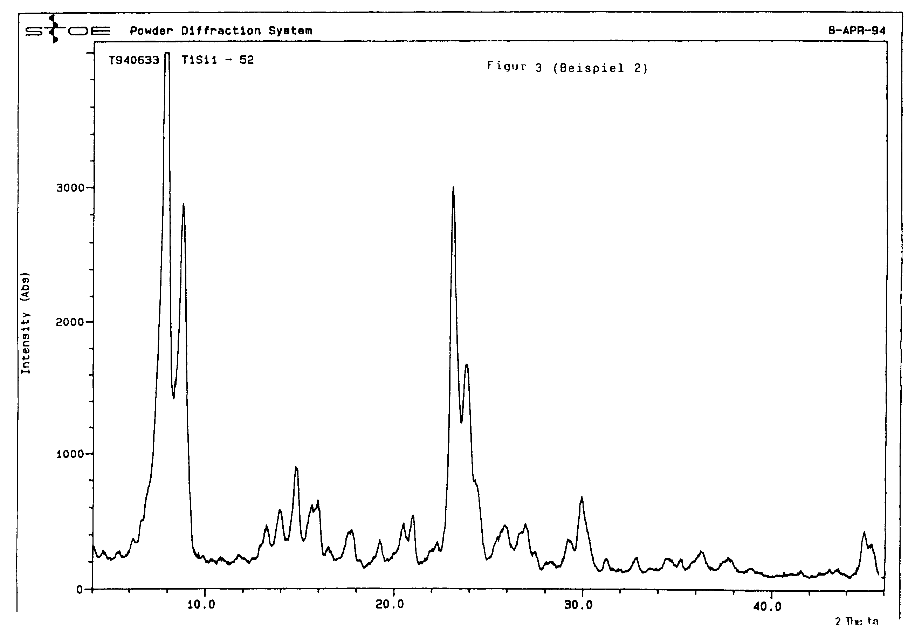

Beispiel 1 wird mit dem Unterschied wiederholt, daß 17,8 g H-ZSM-5 (SiO₂/Al₂O₃ = 150, d₅₀ = 5.7 µm) zum Einsatz kommen.
Naßchemisch bestimmte Zusammensetzung des so hergestellten Katalysators: TiO₂: 2,3 Gew.-%; SiO₂: 96,9 Gew.-%; Al₂O₃: 0,8 Gew.-%.
Röntgendiffraktogramm: Figur 3
IR-Spektrum: Schulter bei 966 cm⁻¹.

### Beispiel 3:

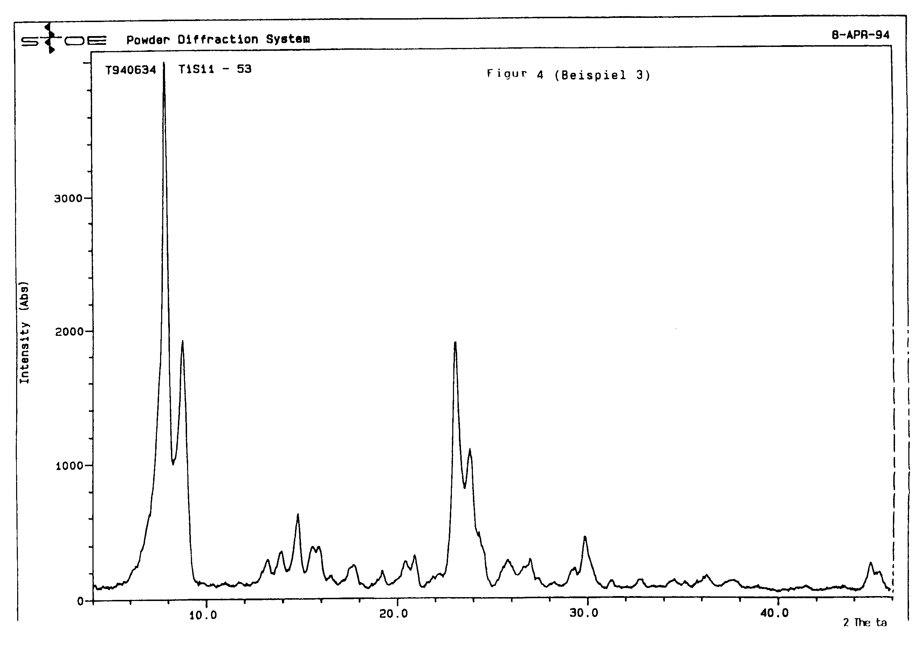

Beispiel 1 wird mit dem Unterschied wiederholt, daß 53,5 g H-ZSM-5 (SiO₂/Al₂O₃ = ca. 1000, d₅₀ = 16.1 µm) zum Einsatz kommen.
Naßchemisch bestimmte Zusammensetzung des so hergestellten Katalysators: TiO₂: 0,6 Gew.-%; SiO₂: 99,3 Gew.-%; Al₂O₃: 0,1 Gew.-%.
Röntgendiffraktogramm: Figur 4 Transmissionselektronenmikroskopisches Schnittbild (Vergrößerung 50.000:1): Figur 7.

### Beispiel 4:

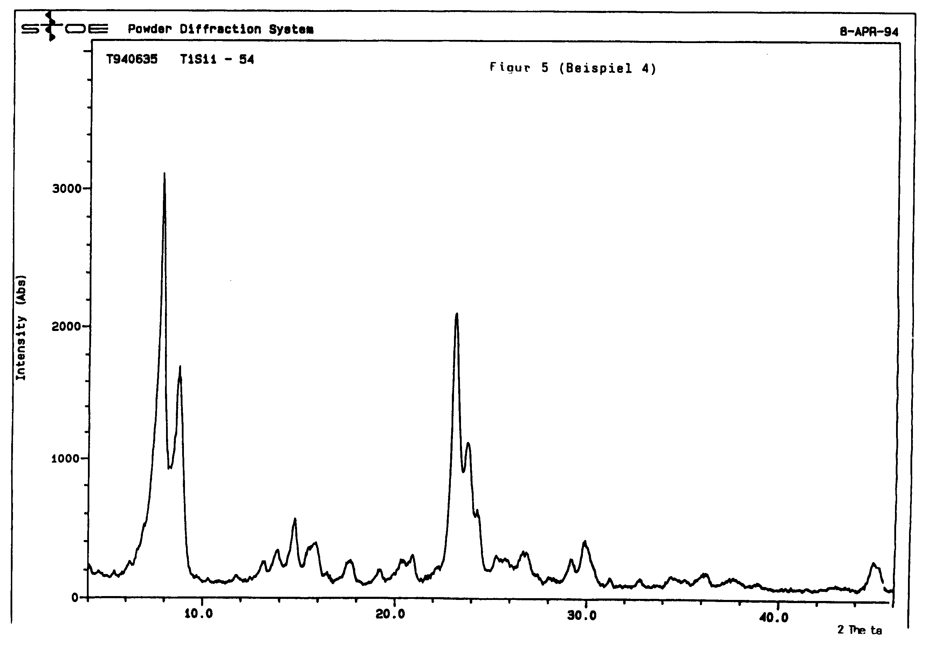

Beispiel 1 wird mitdem Unterschied wiederholt, daß 11,2 g H-ZSM-5 (SiO₂/Al₂O₃ = 28, d₅₀ = 3.7 mm) zum Einsatz kommen. Naßchemisch bestimmte Zusammensetzung des so hergestellten Katalysators: TiO₂: 3,3 Gew.-%; SiO₂: 93,5 Gew.-%; Al₂O₃: 3,2 Gew.-%.
Röntgendiffraktogramm: Figur 5
IR-Spektrum: Bande bei 972 cm⁻¹.

### Beispiel 5 (Vergleichsbeispiel):

Es wurde ein nicht strukturierter, reiner Titansilicalit als Vergleichskatalysator gemäß Beispiel 1 der Patentschrift DE 30 47 798 synthetisiert.
Naßchemisch bestimmte Zusammensetzung des so hergestellten Katalysators: TiO₂: 2,5 Gew.-%; SiO₂: 97,5 Gew.-%.

### Anwendungsbeispiele zur Ammoximierung von Cyclohexanon zu Cyclohexanonoxim

### Beispiel 6:

In einem 100 ml Doppelmantelreaktor mit Druckhaltevorrichtung werden 1.0 g Katalysator gemäß Beispiel 1 in einer Mischung aus 17 ml tert-Butanol und 20.5 ml 14 Gew.-% wässriger Ammoniaklösung vorgelegt. Nach dem Verschließen der Apparatur wird auf 80°C erhitzt und es werden unter Rühren über 5 h gleichzeitig 7.16 ml Cyclohexanon und 7.27 ml 30 Gew.-% wässrige Wasserstoffperoxidlösung zudosiert. Die Mischung wird anschließend 30 min bei 80°C gerührt, dann auf Raumtemperatur abgekühlt, entspannt, mit tert-Butanol verdünnt und filtriert. Nicht umgesetztes Wasserstoffperoxid wird durch iodometrische Titration, nicht umgesetztes Cyclohexanon und gebildetes Cyclohexanonoxim werden gaschromatographisch bestimmt. Bei einem Wasserstoffperoxidumsatz von 99% und einem Cyclohexanonumsatz von 94% wird Cyclohexanonoxim mit > 99% Selelivität, bezogen auf umgesetztes Cyclohexanon, gebildet.

### Beispiel 7:

Beispiel 6 wird mit 1.0 g Katalysator gemäß Beispiel 2 wiederholt. Bei einem Wasserstoffperoxidumsatz von 100% und einem Cyclohexanonumsatz von 79% wird Cyclohexanonoxim mit > 99% Selektivität, bezogen auf umgesetztes Cyclohexanon, gebildet.

### Beispiel 8:

Beispiel 6 wird mit 1.0g Katalysator gemäß Beispiel 3 wiederholt. Bei einem Wasserstoffperoxidumsatz von 99% und einem Cyclohexanonumsatz von 67% wird Cyclohexanonoxim mit > 99% Selektivität, bezogen auf umgesetztes Cyclohexanon, gebildet.

### Beispiel 9:

Beispiel 6 wird mit 1.0g Katalysator gemäß Beispiel 4 wiederholt. Bei einem Wasserstoffperoxidumsatz von 99% und einem Cyclohexanonumsatz von 82% wird Cyclohexanonoxim mit > 99% Selektivität, bezogen auf umgesetztes Cyclohexanon, gebildet.

### Beispiel 10:

Beispiel 6 wird mit 0.66 g Katalysator gemäß Beispiel 5 wiederholt. Bei einem Wasserstoffperoxidumsatz von 100% und einem Cyclohexanonumsatz von 81% wird Cyclohexanonoxim mit 86% Selektivität, bezogen auf umgesetztes Cyclohexanon, gebildet.

### Anwendungsbeispiele zur Epoxidierung von 1-Octen zu 1-Octenoxid mit unbehandeltem Katalysator

### Beispiel 11:

In einem 100 ml Doppelmantelreaktor werden 1.6 g Katalysator gemäß Beispiel 1 in einer Mischung aus 60 g Methanol und 11.6 g 1-Octen vorgelegt. Die Mischung wird auf 55°C erwärmt und unter Rühren mit 3.40 g 49 Gew.-% wässriger Wasserstoffperoxidlösung versetzt. Nach 30 min Rühren bei 55°C wird eine Probe gezogen, filtriert und analysiert. Nicht ungesetztes Wasserstoffperoxid wird durch cerimetrische Titration, nicht umgesetztes 1-Octen und die Oxidationsprodukte 1-Octenoxid, 1-Methoxy-2-octanol und 2-Methoxy-1-octanol werden gaschromatographisch bestimmt.
Bei einem Wasserstoffperoxidumsatz von 29% werden 66% Oxidationsprodukte, bezogen auf umgesetztes Wasserstoffperoxid, gebildet. Die Epoxidselektivität, bezogen auf gebildete Oxidationsprodukte, liegt bei 74%.

### Beispiel 12:

Beispiel 11 wird mit 1.1 g Katalysator gemäß Beispiel 2 wiederholt. Bei einem Wasserstoffperoxidumsatz von 25% werden 78% Oxidationsprodukte, bezogen auf umgesetztes Wasserstotffperoxid, gebildet. Die Epoxidselektivität, bezogen auf gebildete Oxidationsprodukte, liegt bei 72%.

### Beispiel 13:

Beispiel 11 wird mit 4.0 g Katalysator gemäß Beispiel 3 wiederholt. Bei einem Wasserstoffperoxidumsatz von 34% werden 67% Oxidationsprodukte, bezogen auf umgesetztes Wasserstoffperoxid, gebildet. Die Epoxidselelitivität, bezogen auf gebildete Oxidationsprodukte, liegt bei 69%.

### Beispiel 14:

Beispiel 11 wird mit 0.75 g Katalysator gemäß Beispiel 4 wiederholt. Bei einem Wasserstoffperoxidumsatz von 21% werden 53% Oxidationsprodukte bezogen auf umgesetztes Wasserstoffperoxid gebildet. Die Epoxidselektivität, bezogen auf gebildete Oxidationsprodukte, liegt bei 44%.

### Beispiel 15:

Beispiel 11 wird mit 0.66 g Katalysator gemäß Beispiel 5 wiederholt. Bei einem Wasserstoffperoxidumsatz von 21% werden 85% Oxidationsprodukte, bezogen auf umgesetztes Wasserstoffperoxid, gebildet. Die Epoxidselektivität, bezogen auf gebildete Oxidationsprodukte, liegt bei 88%.

### Neutralisation der Katalysatoren mit Natriumacetat

### Beispiel 16:

5 g Katalysator gemäß Beispiel 1 werden mit einer Lösung von 1 g Natriumacetat in 100 ml entionisiertem Wasser 20 min unter Rückfluß erhitzt. Danach wird filtriert, der Katalysator mit heißem, entionisiertem Wasser und mit Methanol gewaschen und an der Luft getrocknet. Eine Suspension von 0.5 g des Katalysators in 10 ml entionisiertem Wasser zeigt vor der Neutralisation einen pH von 3.7 und nach der Neutralisation einen pH von 6.3.

### Beispiel 17:

Beispiel 16 wird mit 5 g Katalysator gemäß Beispiel 2 wiederholt. Eine Suspension von 0.5 g des Katalysators in 10 ml entionisiertem Wasser zeigt vor der Neutralisation einen pH von 3.8 und nach der Neutralisation einen pH von 6.5.

### Beispiel 18:

Beispiel 16 wird mit 5 g Katalysator gemäß Beispiel 3 wiederholt. Eine Suspension von 0.5 g des Katalysators in 10 ml entionisiertem Wasser zeigt vor der Neutralisation einen pH von 4.4 und nach der Neutralisation einen pH von 6.5.

### Beispiel 19:

Beispiel 16 wird mit 5 g Katalysator gemäß Beispiel 4 wiederholt. Eine Suspension von 0.5 g des Katalysators in 10 ml entionisiertem Wasser zeigt vor der Neutralisation einen pH von 3.8 und nach der Neutralisation einen pH von 6.6.

### Beispiel 20:

Beispiel 16 wird mit 5 g Katalysator gemäß Beispiel 5 wiederholt. Eine Suspension von 0.5 g des Katalysators in 10 ml entionisiertem Wasser zeigt vor der Neutralisation einen pH von 5.2 und nach der Neutralisation einen pH von 7.7.

### Anwendungsbeispiele zur Epoxidierung von 1-Octen zu 1-Octenoxid mit neutrallsiertem Katalysator

### Beispiel 21:

Beispiel 11 wird mit 1.6 g Katalysator gemäß Beispiel 16 wiederholt. Bei einem Wasserstoffperoxidumsatz von 30% werden 84% Oxidationsprodukte, bezogen auf umgesetztes Wasserstoffperoxid, gebildet. Die Epoxidselektivität, bezogen auf gebildete Oxidationsprodukte, liegt mit 92% höher, als bei Verwendung des nicht neutralisierten Katalysators aus Beispiel 1.

### Beispiel 22:

Beispiel 11 wird mit 1.1 g Katalysator gemäß Beispiel 17 wiederholt. Bei einem Wasserstoffperoxidumsatz von 29% werden 85% Oxidationsprodukte, bezogen auf umgesetztes Wasserstoffperoxid, gebildet. Die Epoxidselektivität, bezogen auf gebildete Oxidationsprodukte, liegt mit 89% höher, als bei Verwendung des nicht neutralisierten Katalysators gemäß Beispiel 2.

### Beispiel 23:

Beispiel 11 wird mit 4.0 g Katalysator gemäß Beispiel 18 wiederholt. Bei einem Wasserstoffperoxidumsatz von 35% werden 92% Oxidationsprodukte, bezogen auf umgesetztes Wasserstoffperoxid, gebildet. Die Epoxidselektivität, bezogen auf gebildete Oxidationsprodukte, liegt mit 88% höher, als bei Verwendung des nicht neutralisierten Katalysators aus Beispiel 3.

### Beispiel 24:

Beispiel 11 wird mit 0.76 g Katalysator gemäß Beispiel 19 wiederholt. Bei einem Wasserstoffperoxidumsatz von 20% werden 94% Oxidationsprodukte bezogen auf umgesetztes Wasserstoffperoxid gebildet. Die Epoxidselektivität, bezogen auf gebildete Oxidationsprodukte, liegt mit 91% höher, als bei Verwendung des nicht neutralisierten Katalysators aus Beispiel 4.

### Beispiel 25:

Beispiel 11 wird mit 0.66 g Katalysator gemäß Beispiel 20 wiederholt. Bei einem Wasserstoffperoxidumsatz von 26% werden 100% Oxidationsprodukte, bezogen auf umgesetztes Wasserstoffperoxid, gebildet. Die Epoxidselektivität, bezogen auf gebildete Oxidationsprodukte, liegt bei 98%.

## Patentansprüche

1. Katalysator, bestehend aus den Oxiden Von Silicium, Aluminium und Titan, dadurch gekennzeichnet, daß die Katalysatorteilchen aus einem Kern der Zusammensetzung (SiO₂)ₓ(AlO₂)_{y}M_{y} mit x/y = 10 - 75 und M = H, Na, K, NH₄, NR₄, wobei R = C 1-8-Alkyl ist, und einer Schale der Zusammensetzung (SiO₂)ₙ(TiO₂)ₘ mit n/m = 12 - 1000 aufgebaut sind und sowohl Kern als auch Schale eine Kristallstruktur vom Typ MFI oder MEL besitzen.

2. Katalysator nach Anspruch 1, dadurch gekennzeichnet, daß der Kern die Zusammensetzung (SiO₂)ₓ(AlO₂)_{y}M_{y} mit x/y = 10 - 75 und die Schale die Zusammensetzung (SiO₂)ₙ(TiO₂)ₘ mit n/m = 20 - 200 besitzt.

3. Verfahren zur Herstellung des Katalysators nach Anspruch 1 oder 2, dadurch gekennzeichnet, daß man auf bekanntem Wege ein Synthesegel für die Herstellung eines Titansilicalits herstellt, in dieses Synthesegel ein kristallines Alumosilicat einbringt und das Synthesegel auf bekanntem Wege aufarbeitet.

4. Katalysators nach Anspruch 1 oder 2, dadurch gekennzeichnet, daß er nach dem Calcinieren mit einer Base neutralisiert wird, deren pK_{B}-Wert zwischen 0 und 11 liegt, sodaß die wäßrige Suspension des Katalysators nach der Neutralisation einen pH-Wert zwischen 5 und 9 aufweist.

5. Verfahren zur Herstellung eines Epoxids durch Umsetzung eines Olefins mit Wasserstoffperoxid in flüssiger Phase, dadurch gekennzeichnet, daß die Reaktion in Gegenwart eines Katalysators nach Anspruch 1, 2 oder 4 durchgeführt wird.

6. Verfahren zur Herstellung eines Oxims durch Umsetzung eines Ketons mit Wasserstoffperoxid und Ammoniak in flüssiger Phase, dadurch gekennzeichnet, daß die Reaktion in Gegenwart eines Katalysators nach Anspruch 1, 2 oder 4 durchgeführt wird.

## Claims

1. A catalyst consisting of the oxides of silicon, aluminium and titanium, characterised in that the catalyst particles are built up from a core with the composition (SiO₂)ₓ(AlO₂)_{y}M_{y}, where x/y = 10 - 75 and M = H, Na, K, NH₄, NR₄, wherein R = C 1-8-alkyl, and an outer shell with the composition (SiO₂)ₙ(TiO₂)ₘ, where n/m = 12 - 1000, and both the core and the outer shell have a crystal structure of the MFI or MEL type.

2. A catalyst according to claim 1, characterised in that the core has the composition (SiO₂)ₓ(AlO₂)_{y}M_{y}, where x/y = 10 - 75, and the outer shell has the composition (SiO₂)ₙ(TiO₂)ₘ, where n/m = 20 - 200.

3. A process for preparing the catalyst according to claim 1 or 2, characterised in that a synthesis gel for the preparation of a titanium silicalite is prepared in a known manner, a crystalline aluminosilicate is introduced into this synthesis gel and the synthesis gel is worked up in a known manner.

4. A catalyst according to claim 1 or 2, characterised in that, after calcination, it is neutralised with a base whose pK_{B} value is between 0 and 11, so that the aqueous suspension of catalyst has a pH between 5 and 9 after neutralisation.

5. A process for preparing an epoxide by reacting an olefin with hydrogen peroxide in the liquid phase, characterised in that the reaction is performed in the presence of a catalyst according to claim 1, 2 or 4.

6. A process for preparing an oxime by reacting a ketone with hydrogen peroxide and ammonia in the liquid phase, characterized in that the reaction is performed in the presence of a catalyst according to claim 1, 2 or 4.

## Revendications

1. Catalyseur, comprenant les oxydes de silicium, d'aluminium et de titane, caractérisé en ce que les particules de catalyseur sont constituées par un noyau de composition (SiO₂)ₓ(AlO₂)_{y}M_{y} dans laquelle x/y = 10 - 75, M = H, Na, K, NH₄, NR₄ et R = un reste alkyle comportant 1 à 8 atomes de carbone et une coquille de composition (SiO₂)ₙ(TiO₂)ₘ dans laquelle n/m = 12 - 1000 et dans lesquelles tant le noyau que la coquille présentent une structure de cristal de type MFI ou MEL.

2. Catalyseur selon la revendication 1, caractérisé en ce que le noyau présente la composition (SiO₂)ₓ(AlO₂)_{y}M_{y} dans laquelle x/y = 10 - 75 et en ce que la coquille présente la composition (SiO₂)ₙ(TiO₂)ₘ dans laquelle n/m = 20 - 200.

3. Procédé pour la fabrication du catalyseur selon la revendication 1 ou 2, caractérisé en ce qu'on prépare de manière connue un gel de synthèse pour la fabrication d'un silicalite de titane, en ce qu'on introduit dans ce gel de synthèse un aluminosilicate cristallin et en ce qu'on termine le traitement du gel de synthèse de manière connue.

4. Catalyseur selon la revendication 1 ou 2, caractérisé en ce qu'on le neutralise après la calcination avec une base dont la valeur pK_{B} est comprise entre 0 et 11 de telle manière que la suspension aqueuse du catalyseur présente un pH compris entre 5 et 9 après la neutralisation.

5. Procédé pour la fabrication d'un époxyde par réaction d'une oléfine avec du peroxyde d'hydrogène en phase liquide, caractérisé en ce que la réaction est effectuée en présence d'un catalyseur selon la revendication 1, 2 ou 4.

6. Procédé pour la fabrication d'une oxime par réaction d'une cétone avec du peroxyde d'hydrogène et de l'ammoniaque en phase liquide, caractérisé en ce que la réaction est effectuée en présence d'un catalyseur selon la revendication 1, 2 ou 4.
